(19) Europäisches Patentamt
European Patent Office
Office européen des brevets

(11) Veröffentlichungsnummer: **0 224 019 A2**

(12) **EUROPÄISCHE PATENTANMELDUNG**

(21) Anmeldenummer: **86114490.5**

(22) Anmeldetag: **20.10.86**

(51) Int. Cl.⁴: **C07D 231/38**, C07D 401/04, A01N 43/56, A01N 43/40

(30) Priorität: **31.10.85 DE 3538731**

(43) Veröffentlichungstag der Anmeldung: **03.06.87 Patentblatt 87/23**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gehring, Reinhold, Dr.**
**Dasnoeckel 49**
**D-5600 Wuppertal 11(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Santel, Hans-Joachim, Dr.**
**Grünstrasse 9a**
**D-5090 Leverkusen(DE)**
Erfinder: **Schmidt, Robert R., Dr.**
**Im Waldwinkel 110**
**D-5060 Bergisch-Gladbach 2(DE)**
Erfinder: **Lürssen, Klaus, Dr.**
**August-Klerspel-Strasse 151**
**D-5060 Bergisch-Gladbach 2(DE)**

(54) **1-Aryl-4-nitro-pyrazole.**

(57) Die Erfindung betrifft 1-Aryl-4-nitro-pyrazole der Formel (I),

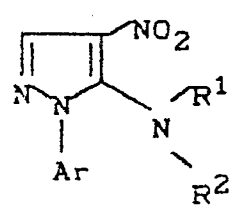

(I)

in welcher
$R^1$ für jeweils gegebenenfalls substituiertes Alkyl, Alkenyl, Alkinyl oder Cycloalkyl steht und
$R^2$ für Wasserstoff, für jeweils gegebenenfalls substituiertes Alkyl, Alkenyl, Alkinyl oder Cycloalkyl oder für einen Rest $-\underset{X}{\overset{\|}{C}}-R^3$ steht, oder
$R^1$ und $R^2$ gemeinsam mit dem Stickstoffatom, an welches sie gebunden sind, für einen gesättigten Heterocyclus stehen, der gegebenenfalls weitere Heteroatome enthalten kann und
Ar für jeweils gegebenenfalls substituiertes Phenyl oder Pyridyl steht,

EP 0 224 019 A2

wobei
X für Sauerstoff oder Schwefel steht und
$R^3$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl, für gegebenenfalls substituiertes Aryl, für gegebenenfalls substituiertes Aryloxy, für gegebenenfalls substituiertes Arylthio, oder für gegebenenfalls substituiertes Arylamino steht,
wobei jedoch
$R^2$ nur dann für Wasserstoff oder für einen Rest $-\underset{X}{\overset{}{\underset{\|}{C}}}-R^3$ steht, wenn
$R^1$ nicht gleichzeitig für unsubstituiertes oder für durch einen Alkoximinorest substituiertes Alkyl steht,
mehrere Verfahren zu ihrer Herstellung und ihre Verwendung als Herbizide und Wachstumsregulatoren.

# 1-Aryl-4-nitro-pyrazole

Die Erfindung betrifft neue 1-Aryl-4-nitro-pyrazole, mehrere Verfahren zu ihrer Herstellung und ihre Verwendung als Herbizide und Pflanzenwachstumsregulatoren.

Es ist bereits bekannt, daß bestimmte substituierte 5-Amino-1-phenyl-pyrazole, wie beispielsweise das 4-Cyano-5-propionamido-1-(2,4,6-trichlorphenyl)-pyrazol herbizide Eigenschaften besitzen (vgl.z.B. DE-OS 3 226 513).

Die herbizide Wirkung dieser bekannten Verbindungen gegenüber Umkräutern ist jedoch ebenso wie ihre Verträglichkeit gegenüber wichtigen Kulturpflanzen nicht immer in allen Anwendungsbereichen völlig zufriedenstellend.

Über eine pflanzenwachstumsregulatorische Wirksamkeit der bekannten Verbindungen ist nichts bekannt.

Es wurden neue 1-Aryl-4-nitro-pyrazole der allgemeinen Formel (I),

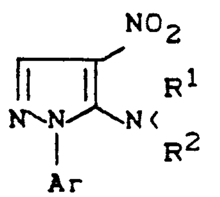

(I)

in welcher

$R^1$ für jeweils gegebenenfalls substituiertes Alkyl, Alkenyl, Alkinyl oder Cycloalkyl steht und

$R^2$ für Wasserstoff, für jeweils gegebenenfalls substituiertes Alkyl, Alkenyl, Alkinyl oder Cycloalkyl oder für einen Rest $-\underset{X}{\overset{\|}{C}}-R^3$ steht,

oder

$R^1$ und $R^2$ gemeinsam mit dem Stickstoffatom, an welches sie gebunden sind, für einen gesättigten Heterocyclus stehen, der gegebenenfalls weitere Heteroatome enthalten kann und

Ar für jeweils gegebenenfalls substituiertes Phenyl oder Pyridyl steht,

wobei

X für Sauerstoff oder Schwefel steht und

$R^3$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl, für gegebenenfalls substituiertes Aryl, für gegebenenfalls substituiertes Aryloxy, für gegebenenfalls substituiertes Arylthio, oder für gegebenenfalls substituiertes Arylamino steht,

wobei jedoch

$R^2$ nur dann für Wasserstoff oder für einen Rest $-\underset{X}{\overset{\|}{C}}-R^3$ steht, wenn

$R^1$ nicht gleichzeitig für unsubstituiertes oder für durch einen Alkoximinorest substituiertes Alkyl steht,

gefunden.

Weiterhin wurde gefunden, daß man die neuen 1-Aryl-4-nitro-pyrazole der Formel (I)

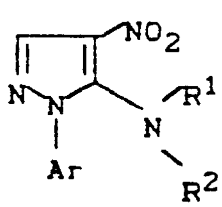

(I)

in welcher

$R^1$ für jeweils gegebenenfalls substituiertes Alkyl, Alkenyl, Alkinyl oder Cycloalkyl steht und

$R^2$ für Wasserstoff, für jeweils gegebenenfalls substituiertes Alkyl, Alkenyl, Alkinyl oder Cycloalkyl oder für

einen Rest $-\underset{X}{\overset{}{\underset{\|}{C}}}-R^3$ steht, oder

$R^1$ und $R^2$ gemeinsam mit dem Stickstoffatom, an welches sie gebunden sind, für einen gesättigten Heterocyclus stehen, der gegebenenfalls weitere Heteroatome enthalten kann und

Ar für jeweils gegebenenfalls substituiertes Phenyl oder Pyridyl steht,

wobei

X für Sauerstoff oder Schwefel steht und

$R^3$ für Wasserstoff, Alkyl, Alkoxy Alkoxyalkyl, Alkylthio, Alkylthioalkyl, Alkylamino, Dialkylamino, Halogenalkyl, Alkenyl, Alkinyl, für gegebenenfalls substituiertes Cycloalkyl, für gegebenenfalls substituiertes Aryl, für gegebenenfalls substituiertes Aryloxy, für gegebenenfalls substituiertes Arylthio oder für gegebenenfalls substituiertes Arylamino steht,

wobei jedoch

$R^2$ nur dann für Wasserstoff oder für einen Rest $-\underset{X}{\overset{}{\underset{\|}{C}}}-R^3$ steht,

wenn $R^1$ nicht gleichzeitig für unsubstituiertes oder für durch einen Alkoximinorest substituiertes Alkyl steht, mit Hilfe der im Folgenden beschriebenen Verfahren erhält:

Man erhält 1-Aryl-4-nitro-pyrazole der Formel (I)

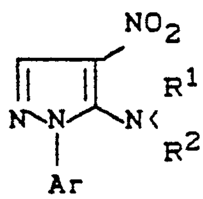

(I)

in welcher

$R^1$, $R^2$ und Ar die oben angegebene Bedeutung haben, wenn man

(a) 5-Halogen-4-nitro-1-aryl-pyrazole der Formel (II)

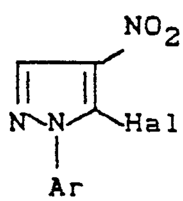

(II)

in welcher

Ar die oben angegebene Bedeutung hat und

Hal für Halogen steht,

mit Aminoverbindungen der Formel (III),

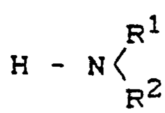

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels umsetzt,

oder wenn man

b) 5-Amino-4-nitro-1-aryl-pyrazole der Formel (Ia),

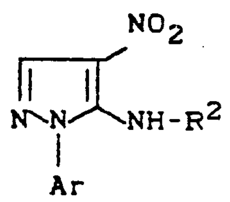

(Ia)

in welcher
$R^2$ und Ar die oben angegebene Bedeutung haben,
mit Alkylierungsmitteln der Formel (IV),
$R^1$ -A (IV)
in welcher
$R^1$ die oben angegebene Bedeutung hat und
A für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt;
oder man erhält
c) 5-Amino-4-nitro-1-aryl-pyrazole der Formel (I b)

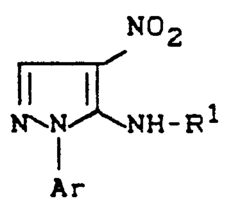

(I b)

in welcher
$R^1$ und Ar die oben angegebene Bedeutung haben,
wenn man
die nach Verfahren (b) erhältlichen 5-Acylamino-4-nitro-1-aryl-pyrazole der Formel (Ic),

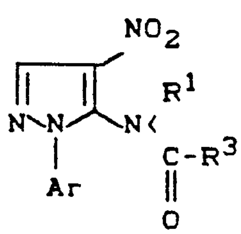

(I c)

in welcher
$R^1$, $R^3$ und Ar die oben angegebene Bedeutung haben,
mit Säuren oder Basen gegebenenfalls in Gegenwart eines Verdünnungsmittels umsetzt;
oder man erhält
d) die substituierten 5-Amino-4-nitro-1-arylpyrazole der Formel (I d)

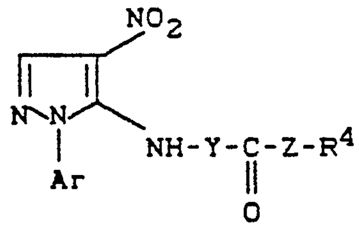

(I d)

in welcher
Ar die oben angegebene Bedeutung hat,
Y für einen zweifach verknüpften Alkylen-, Alkenylen-oder Alkinylenrest steht,
Z für Sauerstoff oder für einen Rest $-\underset{|}{N}-R^5$ steht,
$R^4$ für Alkyl, Alkenyl oder Alkinyl steht oder für den Fall, daß Z für einen -NH-Rest oder einen Alkansulfonamido-Rest steht, auch für Wasserstoff steht und für den Fall, daß Z für Sauerstoff steht auch für ein Äquivalent eines anorganischen Kations oder eines gegebenenfalls substituierten Ammoniumions steht,
$R^5$ für Wasserstoff, Alkyl, Alkenyl, Alkinyl, Alkoxy oder für Alkylsulfonyl steht oder
$R^4$ und $R^5$ gemeinsam mit dem Stickstoffatom, an welches sie gebunden sind, für einen Heterocyclus stehen,
wenn man die nach Verfahren (c) erhältlichen Pyrazolylcarbonsäure-Derivate der Formel (I e)

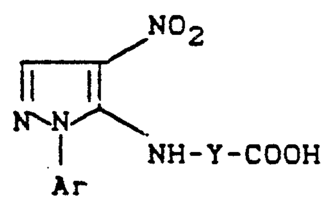

(I e)

in welcher
Ar und Y die oben angegebene Bedeutung haben,
mit Verbindungen der Formel (V),
$R^4$-Z-H (V)
in welcher
Z und $R^4$ die oben angegebene Bedeutung haben,
gegebenenfalls in Gegenwart eines Verdünnungsmittels und gegebenenfalls in Gegenwart eines Reaktionshilfsmittels umsetzt.

Schließlich wurde gefunden, daß die neuen 1-Aryl-4-nitro-pyrazole der Formel (I) herbizide, insbesondere auch selektiv-herbizide und wachstumsregulatorische Eigenschaften besitzen.

Überraschenderweise zeigen die erfindungsgemäßen 1-Aryl-4-nitro-pyrazole der allgemeinen Formel (I) eine bessere herbizide Wirksamkeit gegenüber Problemunkräutern und gleichzeitig eine bessere Selektivität gegenuber wichtigen Kulturpflanzen als die aus dem Stand der Technik bekannten substituierten 5-Amino-1-phenyl-pyrazole, wie beispielsweise das 4-Cyano-5-propionamido-1-(2,4,6-trichlorphenyl)-pyrazol, welche chemisch und wirkungsmäßig naheliegende Verbindungen sind.

Die erfindungsgemäßen 1-Aryl-4-nitro-pyrazole sind durch die Formel (I) allgemein definiert.

Bevorzugt sind Verbindungen der Formel (I), bei welchen
$R^1$ für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils infrage kommen; Halogen, Cyano, Nitro, Hydroxy, geradkettiges oder verzweigtes Alkoxy mit 1 bis 6 Kohlenstoffatomen, Amino, jeweils geradkettiges oder verzweigtes Alkylamino oder Dialkylamino mit jeweils 1 bis 6 Kohlenstoffatomen in den einzelnen Alkylteilen, Carboxy oder ein Rest $-\underset{O}{\overset{}{C}}-Z-R^4$;
$R^1$ weiterhin für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht,
$R^2$ für Wasserstoff, für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils infrage kommen; Halogen, Cyano, Nitro, Hydroxy, geradkettiges oder verzweigtes Alkoxy mit 1 bis 6

Kohlenstoffatomen, Amino oder jeweils geradkettiges oder verzweigtes Alkylamino oder Dialkylamino mit jeweils 1 bis 6 Kohlenstoffatomen in den einzelnen Alkylteilen; $R^2$ weiterhin für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht oder außerdem für einen Rest $-\underset{\underset{X}{\|}}{C}-R^3$ steht oder

$R^1$ und $^2$ gemeinsam mit dem Stickstoffatom, an welches sie gebunden sind, für einen 3-bis 7-gliedrigen gesättigten Heterocyclus stehen, der bis zu 2 weitere Heteroatome, insbesondere Stickstoff, Sauerstoff und/oder Schwefel enthalten kann und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_n-R^6$, wobei

X für Sauerstoff oder Schwefel steht,

$R^3$ für Wasserstoff, für jeweils geradkettiges oder verzweigtes Alkyl, Alkoxy, Alkoxyalkyl, Alkylthio, Alkylthioalkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen, für jeweils geradkettiges oder verzweigtes Alkenyl oder Alkinyl mit jeweils 2 bis 4 Kohlenstoffatomen, außerdem für gegebenenfalls einfach oder mehrfach, gleich oder verschieden durch Halogen, Alkyl oder Halogenalkyl mit jeweils 1 bis 4 Kohlenstoffatomen und im Fall des Halogenalkyl mit 1 bis 5 Halogenatomen substituiertes Cycloalkyl mit 3 bis 7 Kohlenstoffatomen, sowie für jeweils gegebenenfalls einfach oder mehrfach gleich oder verschieden substituiertes Phenyl, Phenoxy, Phenylthio oder Phenylamino steht, wobei als Phenylsubstituenten jeweils infrage kommen: Halogen, jeweils geradkettiges oder verzweigtes Alkyl, Alkoxy oder Halogenalkyl mit jeweils 1 bis 4 Kohlenstoffatomen und im Fall des Halogenalkyl mit 1 bis 9 gleichen oder verschiedenen Halogenatomen,

Z für Sauerstoff oder einen Rest $-\underset{|}{N}-R^5$ steht,

$R^4$ für jeweils geradkettiges oder verzweigtes Alkyl, Alkenyl oder Alkinyl mit jeweils bis zu 6 Kohlenstoffatomen steht oder für den Fall, daß Z für einen -NH-Rest oder einen Alkansulfonamido-Rest steht, auch für Wasserstoff steht oder für den Fall, daß Z für Sauerstoff steht auch für ein Äquivalent eines Alkali-, Erdalkali-, Übergangsmetall-oder eines gegebenenfalls durch Alkyl mit 1 bis 18 Kohlenstoffatomen, Benzyl oder Phenethyl substituierten Ammoniumions steht und

$R^5$ für Wasserstoff, für jeweils geradkettiges oder verzweigtes Alkyl, Alkenyl oder Alkinyl mit jeweils bis zu 6 Kohlenstoffatomen oder für jeweils geradkettiges oder verzweigtes Alkoxy oder Alkylsulfonyl mit jeweils 1 bis 6 Kohlenstoffatomen steht, oder

$R^4$ und $R^5$ gemeinsam mit dem Stickstoffatom, an welches sie gebunden sind, für einen 3-bis 7-gliedrigen gesättigten Heterocyclus stehen, der bis zu 2 weitere Heteroatome, insbesondere Stickstoff, Sauerstoff und/oder Schwefel enthalten kann,

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

n für eine Zahl 0, 1 oder 2 steht,

wobei jedoch $R^2$ nur dann für Wasserstoff oder für einen Rest $-\underset{\underset{X}{\|}}{C}-R^3$ steht, wenn $R^1$ nicht gleichzeitig für unsubstituiertes oder für durch einen Alkoximinorest substituiertes Alkyl steht.

Halogen steht in der bevorzugten Definition von Verbindungen der Formel (I) jeweils für Fluor, Chlor, Brom oder Iod.

Besonders bevorzugt sind Verbindungen der Formel (I), bei welchen

$R^1$ für jeweils gegebenenfalls ein-bis fünffach, gleich oder verschieden substituiertes Methyl, Ethyl, n-oder i-Propyl, n-, i-, s-oder t-Butyl, Allyl, Butenyl, Butinyl oder Propargyl steht, wobei als Substituenten jeweils infrage kommen: Fluor, Chlor, Brom, Iod, Cyano, Nitro Hydroxy, Methoxy, Ethoxy, n-oder i-Propoxy, Amino, Methylamino, Ethylamino, Diamethylamino, Diethylamino oder Di-n-propylamino, Carboxy oder ein Rest $-\underset{\underset{O}{\|}}{C}-Z-R^4$; $R^1$ weiterhin für jeweils gegebenenfalls ein-bis fünffach, gleich oder verschieden durch Chlor, Brom, Methyl oder Ethyl substituiertes Cyclopropyl, Cyclopentyl, Cyclohexyl oder Cycloheptyl steht,

$R^2$ für Wasserstoff, für jeweils gegebenenfalls ein-bis dreifach, gleich oder verschieden substituiertes Methyl, Ethyl, n-oder i-Propyl, n-, i-, s-oder t-Butyl, Allyl, Butenyl, Propargyl oder Butinyl steht, wobei als Substituenten jeweils infrage kommen: Fluor, Chlor, Brom, Iod, Cyano, Nitro, Hydroxy, Methoxy, Ethoxy, n-oder i-Propoxy, Amino, Methylamino, Ethylamino, Dimethylamino, Diethylamino oder Di-n-propylamino; $R^2$ weiterhin für jeweils gegebenenfalls ein-bis fünffach, gleich oder verschieden durch Chlor, Brom, Methyl

oder Ethyl substituiertes Cyclopropyl, Cyclopentyl, Cyclohexyl oder Cycloheptyl steht oder auch für einen Rest $-\underset{X}{\overset{}{\underset{\|}{C}}}-R^3$ steht oder

$R^1$ und $R^2$ gemeinsam mit dem Stickstoffatom, an welches sie gebunden sind, für Pyrrolidinyl, Piperidinyl, Perhydroazepinyl oder Morpholinyl stehen, und

Ar für gegebenenfalls ein-bis fünffach, gleich oder verschieden substituiertes Phenyl oder für gegebenenfalls ein-bis vierfach, gleich oder verschieden substituiertes 2-Pyridyl steht, wobei als Substituenten jeweils infrage kommen:

Cyano, Nitro, Fluor, Chlor, Brom, Iod, Methyl, Ethyl, n-und i-Propyl, n-, i-, s-und t-Butyl Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_n-R^6$,

wobei

X für Sauerstoff oder Schwefel steht und

$R^3$ für Wasserstoff, Methyl, Ethyl, n-und i-Propyl, Methoy, Ethoxy, Methoxymethyl, Ethoxymethyl, Methoxyethyl, Ethoxyethyl, Methylthio, Ethylthio, Methylthiomethyl, Methylamino, Ethylamino, Dimethylamino, Allyl, Propargyl, Butenyl, Trifluormethyl, Trichlormethyl, Dichlorfluormethyl, Difluorchlormethyl, Chlormethyl, Iodmethyl, Brommethyl, Dichlormethyl, 1-Chlorethyl, 2-Chlorethyl, 2-Bromethyl, Heptafluor-n-propyl, für jeweils gegebnenfalls ein-bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

Z für Sauerstoff oder einen Rest $-\underset{|}{N}-R^5$ steht,

$R^4$ für Methyl, Ethyl, n-oder i-Propyl, n-, i-, s-oder t-Butyl, Allyl oder Propargyl steht, oder für den Fall, daß Z für einen -NH-Rest oder einen Alkansulfonamido-Rest steht, auch für Wasserstoff steht oder, für den Fall, daß Z für Sauerstoff steht, auch für ein Äquivalent eines Natrium-, Kalium-, Calcium-, Eisen-, Kupfer-, Nickel-, Zinn-, Magnesium-, Zink-, Mangan-, Barium-, Cobalt-oder Ammonium-bzw. Benzylammonium-bzw. Alkylammoniumsalzes steht, wobei Alkyl für Methyl, Ethyl, n-oder i-Propyl, n-, i-, s-oder t-Butyl, n-oder i-Pentyl, n-oder i-Hexyl, n-oder i-Nonyl, n-oder i-Dodecyl steht,

$R^5$ für Wasserstoff, Methyl, Ethyl, n-oder i-Propyl, n-, i-, s-oder t-Butyl, Allyl, Propargyl, Methoxy, Ethoxy, n-oder i-Propoxy, Methylsulfonyl oder Ethylsulfonyl steht, oder

$R^4$ und $R^5$ gemeinsam mit dem Stickstoffatom, an welches sie gebunden sind, für Pyrrolidinyl, Piperidi nyl, Perhydroazepinyl, Piperazinyl oder Morpholinyl stehen und

$R^6$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht und

n für eine Zahl 0, 1 und 2 steht

wobei jedoch

$R^2$ nur dann für Wasserstoff oder für einen Rest $-\underset{X}{\underset{\|}{C}}-R^3$ steht, wenn $R^1$ nicht gleichzeitig für unsubstituiertes oder für durch einen Alkoximinorest substituiertes Alkyl steht.

Ganz besonders bevorzugt sind Verbindungen der Formel (I), bei welchen

$R^1$ für jeweils gegebenenfalls ein-bis dreifach, gleich oder verschieden substituiertes Methyl, Ethyl, n-oder i-Propyl, n-, i-, s-oder t-Butyl, Allyl, Butenyl, Butinyl oder Propargyl steht, wobei als Substituenten jeweils infrage kommen: Fluor, Chlor, Brom, Iod, Cyano, Nitro, Hydroxy, Methoxy, Ethoxy,n-oder i-Propoxy, Amino, Methylamino, Ethylamino, Dimethylamino, Diethylamino oder Di-n-propylamino, Carboxy oder ein Rest $-\underset{O}{\underset{\|}{C}}-Z-R^4$, und außerdem für jeweils gegbenenfalls ein-bis fünffach, gleich oder oder verschieden durch Chlor, Brom, Methyl oder Ethyl substituiertes Cyclopropyl, Cyclopentyl, Cyclohexyl oder Cycloheptyl steht,

$R^2$ für Wasserstoff, für jeweils gegebenenfalls ein-bis dreifach, gleich oder verschieden substituiertes Methyl, Ethyl, n-oder i-Propyl, n-, i-, s-oder t-Butyl, Allyl, Butenyl, Propargyl oder Butinyl steht, wobei als Substituenten jeweils infrage kommen: Fluor, Chlor, Brom, Iod, Cyano, Nitro, Hydroxy, Methoxy, Ethoxy, n-oder i-Propoxy, Amino, Methylamino, Ethylamino, Dimethylamino, Diethylamino oder Di-n-propylamino; außerdem für jeweils gegebenenfalls ein-bis fünffach, gleich oder verschieden durch Chlor, Brom, Methyl oder Ethyl substituiertes Cyclopropyl, Cyclopentyl, Cyclohexyl oder Cycloheptyl steht oder für einen Rest

$-\underset{\overset{\|}{X}}{C}-R^3$ steht oder

$R^1$ und $R^2$ gemeinsam mit dem Stickstoffatom, an welches sie gebunden sind, für Pyrrolidinyl, Piperidinyl, Perhydroazepinyl oder Morpholinyl stehen und

Ar für einen Rest

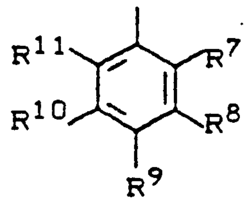

oder für einen Rest

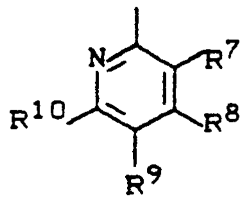

steht,

wobei

X für Sauerstoff oder Schwefel steht und

$R^3$ für Wasserstoff, Methyl, Ethyl, n-und i-Propyl, Methoxy, Ethoxy, Methoxymethyl, Ethoxymethyl, Methoxyethyl, Ethoxyethyl, Methylthio, Ethylthio, Methylthiomethyl, Methylamino, Ethylamino, Dimethylamino, Allyl, Propargyl, Butenyl, Trifluormethyl, Trichlorethyl, Dichlor-fluorethyl, Difluorchlorethyl, Chlormethyl, Iodmethyl, Brommethyl, Dichlormethyl, 1-Chlor-ethyl, 2-Chlorethyl, 2-Bromethyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cy clohexyl, sowie für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

Z für Sauerstoff oder einen Rest $-\underset{|}{N}-R^5$ steht,

$R^4$ für Methyl, Ethyl, n-oder i-Propyl, n-, i-, s-oder t-Butyl, Allyl oder Propargyl steht, oder für den Fall, daß Z für einen -NH-Rest oder für einen Alkansulfonamido-Rest steht, auch für Wasserstoff steht oder, für den Fall, daß Z für Sauerstoff steht auch für ein Äquivalent eines Natrium-, Kalium-, Calcium-, Eisen-, Kupfer-, Nickel-, Zinn-, Zink-, Magnesium-, Mangan-, Barium-, Cobalt-oder Ammonium-bzw. Benzylammonium-bzw. Alkylammoniumsalzes steht, wobei Alkyl für Methyl, Ethyl, n-oder i-Propyl, n-, i-, s-oder t-Butyl, n-oder i-Pentyl, n-oder i-Hexyl, n-oder i-Nonyl, n-oder i-Dodecyl steht,

$R^5$ für Wasserstoff, Methyl, Ethyl, n-oder i-Propyl, n-, i-, s-oder t-Butyl, Allyl, Propargyl, Methoxy, Ethoxy, n-oder i-Propoxy, Methylsulfonyl oder Ethylsulfonyl steht oder

$R^4$ und $R^5$ gemeinsam mit dem Stickstoffatom, an welches sie gebunden sind, für Pyrrolidinyl, Piperi dinyl, Perhydroazepinyl, Piperazinyl oder Morpholinyl stehen,

$R^6$ für Trifluormethyl, Fluordichlormethyl, Difluorchlormethyl, Trichlormethyl, Trichlorethyl, für Methyl oder Ethyl steht,

$R^7$ für Fluor, Chlor, Brom, Methyl oder Ethyl steht,

$R^8$, $R^{10}$ und $R^{11}$ unabhängig voneinander jeweils für Wasserstoff, Fluor, Chlor, Brom, Methyl oder Ethyl stehen,

$R^9$ für Cyano, Nitro, Fluor, Chlor, Brom, Methyl, Ethyl, n-und i-Propyl, n-, i-, s-und t-Butyl, Methoxy, Ethoxy, n-und i-Propoxy, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^6$ steht, wobei n und $R^6$ die oben angegebene Bedeutung haben, und

n für eine Zahl 0, 1 und 2 steht,

wobei jedoch $R^2$ nur dann für Wasserstoff oder für einen Rest $-\overset{\overset{X}{\|}}{C}-R^3$ steht, wenn $R^1$ nicht gleichzeitig für unsubstituiertes oder für durch einen Alkoximinorest substituiertes Alkyl steht.

Im einzelnen seien außer den bei den Herstellungsbeispielen genannten Verbindungen die in der folgenden Tabelle aufgeführten 1-Aryl-4-nitro-pyrazole der allgemeinen Formel (I) genannt:

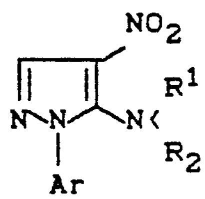

(I)

Tabelle 1

| $-N\langle^{R^1}_{R^2}$ | Ar |
|---|---|
| $CH_3$<br>\|<br>$-NH-CH-COOC_2H_5$ | 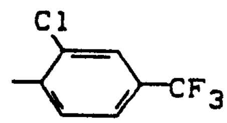 |
| $CH_3$<br>\|<br>$-NH-CH-COOCH_3$ | 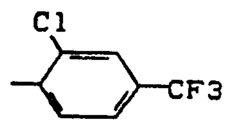 |
| $CH_3$<br>\|<br>$-NH-CH-COOCH_3$ | 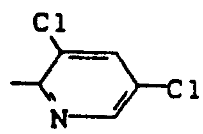 |
| $CH_3$<br>\|<br>$-NH-CH-COOC_2H_5$ | 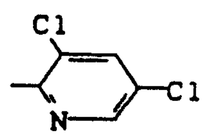 |
| $CH_3$<br>\|<br>$-NH-CH-CO-NH-CH_3$ | 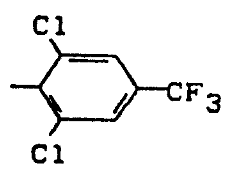 |
| $CH_3$<br>\|<br>$-NH-CH-CO-NH-SO_2-CH_3$ | 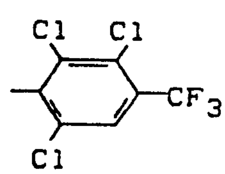 |

Tabelle 1 (Fortsetzung)

| 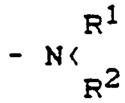 | Ar |
|---|---|
| 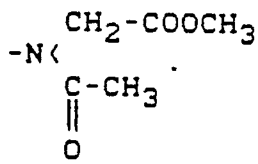 | 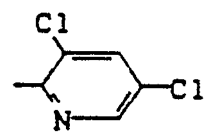 |
| $-NH-CH_2-COOH$ | 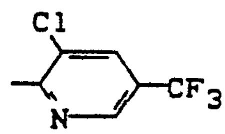 |
| $-NH-CH_2-CO-NH-C_2H_5$ | 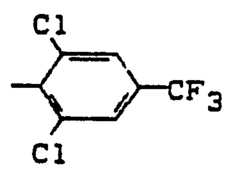 |
| $-NH-CH_2-CO-NH-SO_2-CH_3$ | 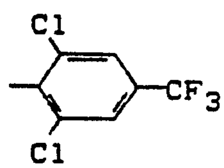 |
| 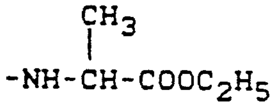 | 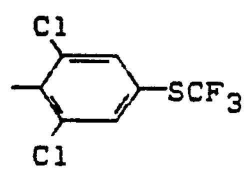 |
| $-NH-CH_2-COOH$ | 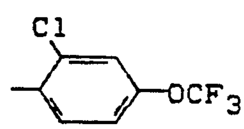 |

Tabelle 1 (Fortsetzung)

| $-N\langle^{R^1}_{R^2}$ | Ar |
|---|---|
| $-NH-CH_2-CH_2-CN$ | 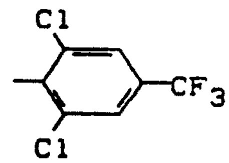 |
| $-NH-\overset{CH_3}{\overset{\vert}{C}}H-CH_2-CN$ | 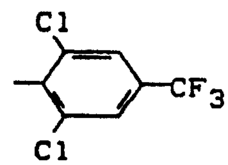 |
| $-NH-CH_2-CH_2-COOC_2H_5$ | 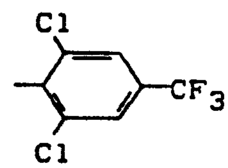 |
| $-NH-CH_2-\overset{CH_3}{\overset{\vert}{C}}H-COOC_2H_5$ | 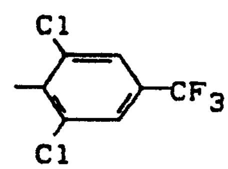 |
| $-NH-CH_2-CN$ | 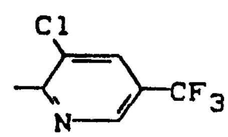 |

Tabelle 1 (Fortsetzung)

| $-N\langle^{R^1}_{R^2}$ | Ar |
|---|---|
| $-NH-CH_2-CO-NH-(CH_2)_2-CH_3$ | 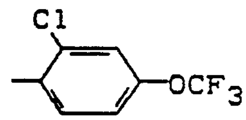 |
| $CH_2-COOCH_3$<br>$-N\langle$<br>$C-CH_3$<br>$\Vert$<br>$O$ | 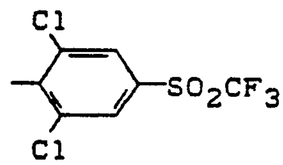 |
| $-NH-CH_2-COOH$ | 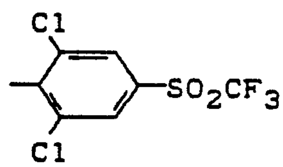 |
| $-NH-CH_2-COO^-Na^+$ | 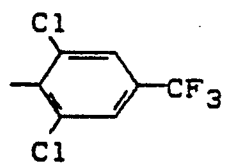 |
| $-NH-CH_2-COO-CH(CH_3)_2$ | 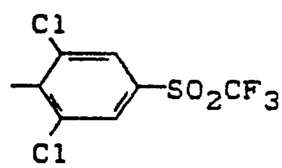 |
| $-NH-CH_2-CN$ | 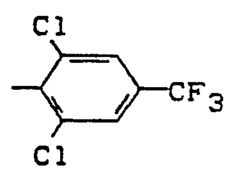 |

Tabelle 1 (Fortsetzung)

| $-N\langle^{R^1}_{R^2}$ | Ar |
|---|---|
| $-NH-CH_2-CH_2-COOCH_3$ | 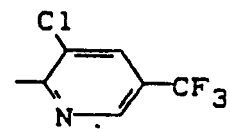 |
| $-NH-CH_2-CH_2-OCH_3$ | 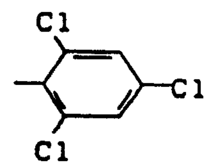 |
| $-NH-CH_2-CH_2-OCH_3$ | 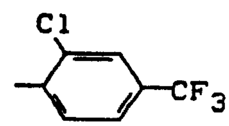 |
| $-NH-CH_2-CH_2-OH$ | 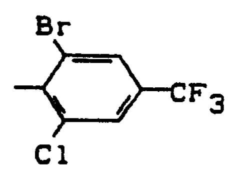 |
| $-NH-CH_2-CH_2-OC_2H_5$ | 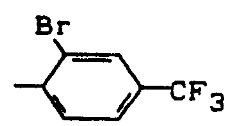 |
| $-N\langle^{CH_3}_{CH_2-CH_2-OCH_3}$ | 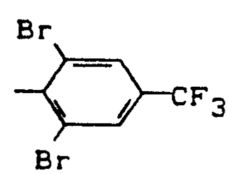 |

Tabelle 1 (Fortsetzung)

| $-N{<}^{R^1}_{R_2}$ | Ar |
| --- | --- |
| $-N{<}^{CH_3}_{CH_3}$ | 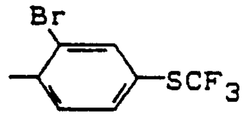 |
| $-N{<}^{CH_3}_{CH_3}$ | 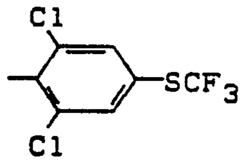 |
| 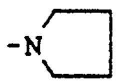 | 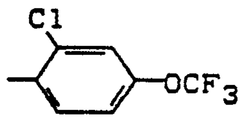 |
| 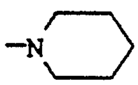 | 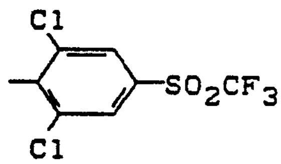 |
| 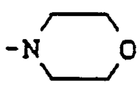 | 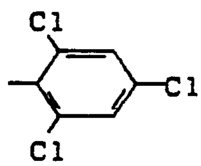 |
| 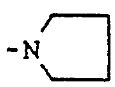 | 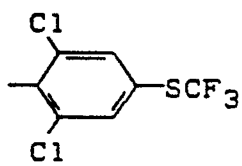 |

Verwendet man als Ausgangsstoffe biespielsweise 5-Chlor-4-nitro-1-(2,4,6-trichlorphenyl)-pyrazol und 2-Methoxyethylamin, so läßt sich der Reaktionsablauf des erfindungsgemäßen Verfahrens (a) durch das folgende Formelschema darstellen:

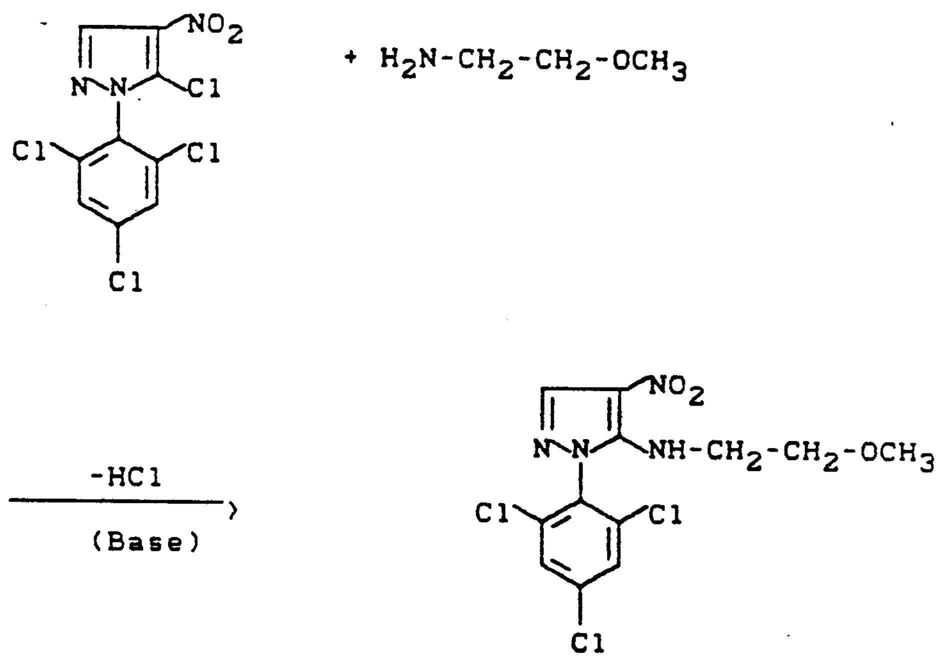


Verwendet man beispielsweise 5-Propionamido-4-nitro-1-(2,6-dichlor-4-trifluormethyl-phenyl)-pyrazol und α-Brompropionsäureethylester als Ausgangsstoffe, so läßt sich der Reaktionsablauf des erfindungsgemäßen Verfahrens (b) durch das folgende Formelschema darstellen:

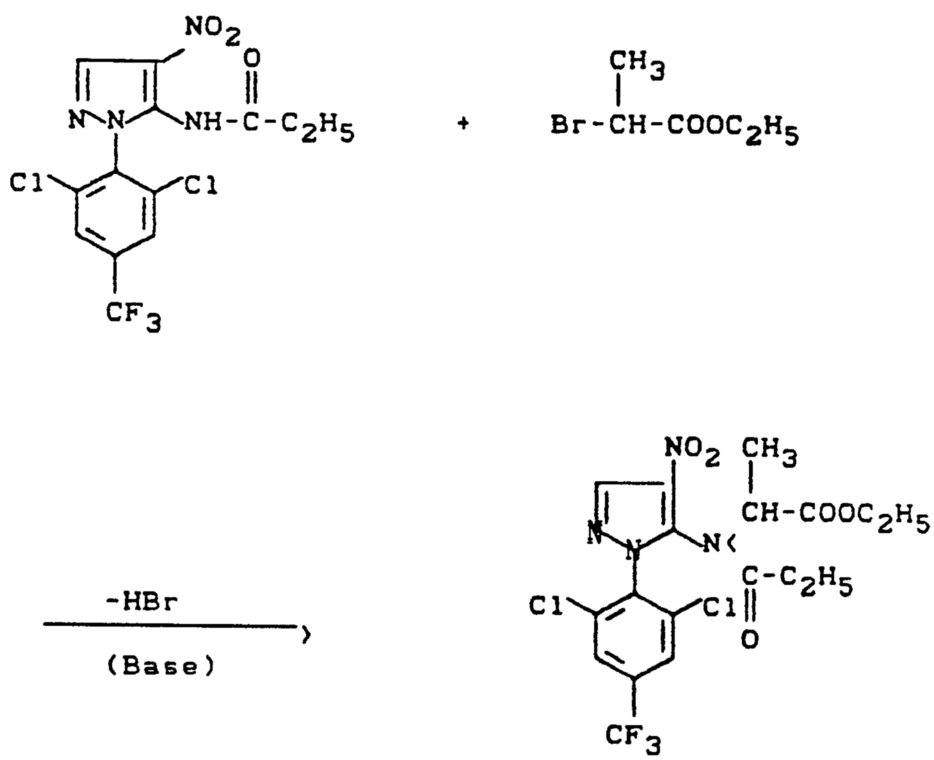


Verwendet man beispielsweise 5-(N-Methoxycarbonylmethyl)-acetamido-4-nitro-1-(3-chlor-5-trifluormethylpyrid-2-yl)-pyrazol als Ausgangsverbindung, so läßt sich der Reaktionsablauf des erfindungsgemäßen Verfahrens (c) durch das folgende Formelschema darstellen:

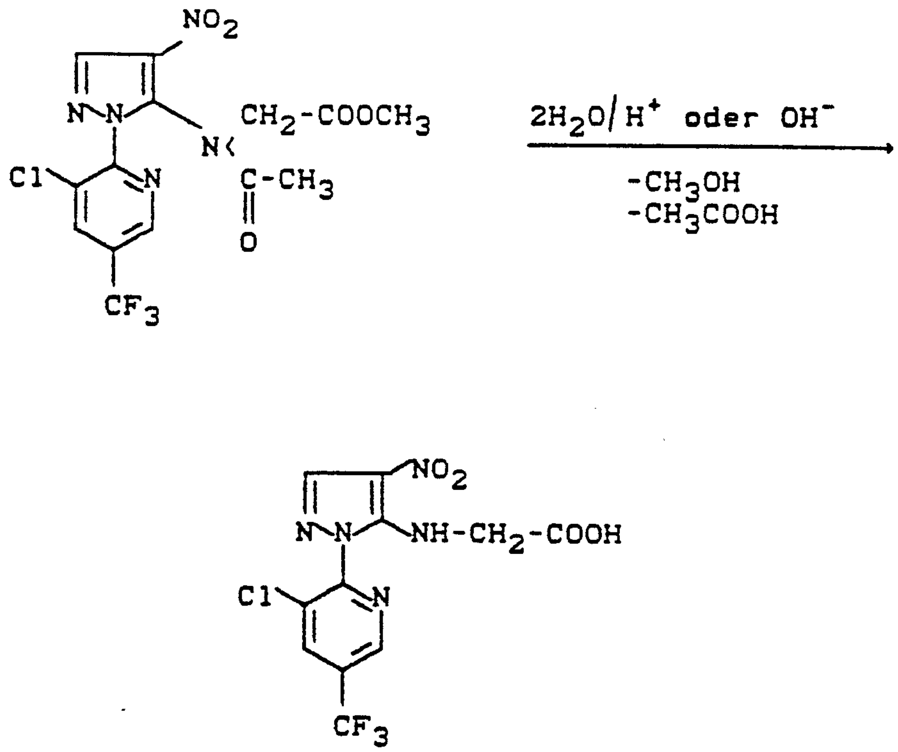


Verwendet man beispielsweise N-[4-Nitro-1-(2,6-dichlor-4-trifluormethylthio-phenyl)-pyrazol-5-yl]-aminoessigsäure und Propargylalkohol als Ausgangsstoffe und Thionylchlorid als Reaktionshilfsmittel, so läßt sich der Reaktionsablauf des erfindungsgemäßen Verfahrens (d) durch das folgende Formelschema darstellen:

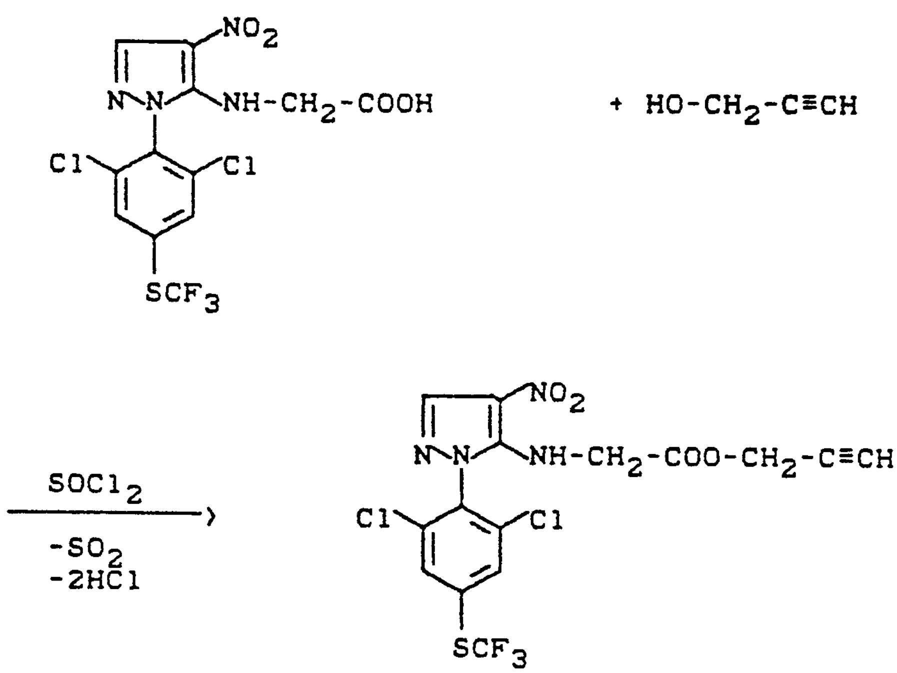


Die zur Durchführung des erfindungsgemäßen Verfahrens (a) als Ausgangsstoffe benötigten 5-Halogen-4-nitro-1-aryl-pyrazole sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diese Substituenten genannt wurden. Hal steht vorzugsweise für Chlor oder Brom.

Die 5-Halogen-4-nitro-1-aryl-pyrazole der Formel (II) sind noch nicht bekannt. Sie sind jedoch Gegenstand der eigenen vorgängigen noch nicht veröffentlichten Patentanmeldungen DE-P 3 501 323 vom 17.01.1985 und DE-P 3 520 330 vom 07.06.1985.

Man erhält sie beispielsweise, wenn man 5-Amino-1-aryl-pyrazole der Formel (VI),

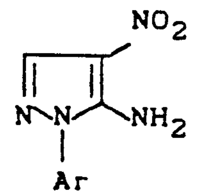

(VI)

in welcher
Ar die oben angegebene Bedeutung hat,
mit Nitritverbindungen der Formel (VII),
$R^{12}$-O-N = O (VII)
in welcher
$R^{12}$ für Wasserstoff, Alkyl oder für ein Alkalimetallkation steht,
in Gegenwart einer Halogenwasserstoffsäure wie beispielsweise Chlorwasserstoffsäure oder Bromwasserstoffsäure oder in Gegenwart eines Haloforms wie beispielsweise Chloroform oder Bromoform bei Temperaturen zwischen -20 °C und +80 °C in üblicher Art und Weise diazotiert (vgl. z.B. "Organikum" 15. Auflage VEB Deutscher Verlag der Wissenschaften, Berlin 1981, S. 652 ff;J. Chem. Soc. C, 1966. 1249 oder Rev. Latinoam. Quim. 13, 100-102 [1982]).

Die 5-Amino-1-aryl-pyrazole der Formel (VI) sind teilweise bekannt (vgl. z.B. DE-OS 3 402 308) teilweise sind sie Gegenstand der eigenen noch nicht vorveröffentlichten Patentanmeldung DE-P 3 520 330 vom 07.06.1985.

Man erhält sie beispielsweise, wenn man Aryl-hydrazine der Formel (VIII),
Ar-NH-$NH_2$ (VIII)
in welcher
Ar die oben angegebene Bedeutung hat,
mit 2-Halogenacrylnitrilen der Formel (IX),

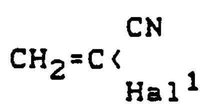

(IX)

in welcher
Hal' für Halogen, insbesondere für Chlor oder Brom steht,
entweder zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispielsweise Natriumacetat bei Temperaturen zwischen -20 °C und +20 °C umsetzt zu den Arylhydrazin-Derivaten der Formel (X),

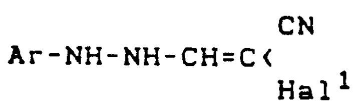

(X)

in welcher
Ar und Hal' die oben angegebenene Bedeutung haben
und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50°C und +150 °C cyclisiert, oder direkt in einem Reaktionsschritt, ohne Isolierung der Zwischenstufe der Formel (X) gegebenenfalls in

Gegenwart eines Verdünnungsmittels wie beispielsweise Ethylenglykolmonoethylether oder Ethanol bei Temperaturen zwischen +50 °C und +150 °C cyclisiert und die so erhältlichen 4-unsubstituierten 5-Aminopyrazole oder Formel (XI),

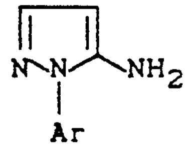

(XI)

in welcher
Ar die oben angegebene Bedeutung hat,
in einer Folgereaktion mit einem Nitrierungsmittel wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20 °C und +50 °C nitriert. Dabei kann es gegebenenfalls von Vorteil sein, vor der Nitrierungsreaktion die Aminogruppe in der 5-Position des Pyrazolringes mit Hilfe üblicher Schutzgruppentechnik, beispielsweise durch Acylierung zu schützen und die Aminoschutzgruppe nach erfolgter Nitrierung ebenfalls in üblicher Art und Weise beispielsweise durch Verseifung mit wässriger oder alkoholischer Base wieder abzuspalten.

Die Arylhydrazine der Formel (VIII) sind bekannt (vgl. z.B. US-PS 4 127 575; US-PS 3 609 158; DE-OS 2 558 399; J. Chem. Soc. C, 1971, 167-174) oder können nach bekannten Verfahren in einfacher analoger Weiser erhalten werden (vgl. z.B. Houben-Weyl "Methoden der organischen Chemie" Band X/2, S. 203, Thieme Verlag Stuttgart, 1967), in dem man beispielsweise die bekannten Aniline bzw. Pyridylamine der Formel (XII),

$Ar\text{-}NH_2$ (XII)

in welcher
Ar die oben angegebene Bedeutung hat,
mit Natriumnitrit in Gegenwart einer Säure wie beispielsweise Schwefelsäure, und dann mit Zinn-II-chlorid ebenfalls in Gegenwart einer Säure wie beispielsweise Salzsäure bei Temperaturen zwischen -20 °C und +80 °C umsetzt oder wenn man Halogenaromaten der Formel (XIII),

$Ar\text{-}Hal^2$ (XIII)

in welcher
Ar die oben angegebene Bedeutung hat und
$Hal^2$ für Halogen, insbesondere für Fluor, Chlor oder Brom steht,
mit Hydrazinhydrat gegebenenfalls in Gegenwart eines Verdünnungsmittels wie beispielsweise Pyridin oder Dioxan bei Temperaturen zwischen 0 °C und 150 °C umsetzt.

Die Nitritverbindungen der Formel (VII), die Arylhydrazine der Formel (VIII), die 2-Halogenacrylnitrile der Formel (IX), die Aniline und Pyridylamine der Formel (XII) und die Halogenaromaten der Formel (XIII) sind allgemein bekannte Verbindungen der organischen Chemie.

Die zur Durchführung des erfindungsgemäßen Verfahrens (a) weiterhin als Ausgangsstoffe benötigten Aminoverbindungen sind durch die Formel (III) allgemein definiert. In dieser Formel (III) stehen $R^1$ und $R^2$ vorzugs weise für diejenigen Reste, die bereits im Zusammenhang mit der Beschreibung der erfindungsgemäßen Stoffe der Formel (I) als bevorzugt für diese Substituenten genannt wurden. Die Aminoverbindungen der Formel (III) sind allgemein bekannte Verbindungen der organischen Chemie.

Die zur Durchführung des erfindungsgemäßen Verfahrens (b) als Ausgangsstoffe benötigten 5-Amino-4-nitro-1-aryl-pyrazole sind durch die Formel (I a) allgemein definiert. In dieser Formel (I a) stehen $R^2$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-4-nitro-1-aryl-pyrazole der Formel (I a) sind teilweise bekannt (vgl.DE-OS 3 402 308), teilweise sind sie Gegenstand der eigenen vorgängigen Patentanmeldung DE-P 3520 330 vom 07.06.1985 und teilweise sind sie erfindungsgemäße Verbindungen und erhältlich mit Hilfe der erfindungsgemäßen Verfahren (a), (b) oder (c).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^1$ vorzugsweise für diejenigen Reste, die bereits im Zusammenhang mit der Beschreibung der erfindungsgemäßen Stoffe der Formel (I) als bevorzugt für diese Substituenten genannt wurden. A steht vorzugsweise für Halogen, insbesondere für Chlor, Brom oder Iod, oder für jeweils gegebenenfalls substituiertes Alkylsulfonyloxy, Alkoxysulfonyloxy oder Arylsulfonyloxy, wie beispielsweise Methansulfonyloxy, Methoxysulfonyloxy oder p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten 5-Acylamino-4-nitro-1-aryl-pyrazole sind durch die Formel (I c) allgemein definiert. In dieser Formel (I c) stehen $R^1$, $R^3$ und Ar vorzugsweise für diejenigen Reste, die bereits im Zusammenhang mit der Beschreibung der erfindungsgemäßen Stoffe der Formel (I) als bevorzugt für diese Substituenten genannten wurden.

Die 5-Acylamino-4-nitro-1-aryl-pyrazole der Formel (I c) sind teilweise baknnt (vgl. DE-OS 3 402 308) teilweise sind sie Gegenstand der eigenen vorgängigen Patentanmeldung DE-P 3 520 330 vom 07.06.1985 und teilweise sind sie erfindungsgemäße Verbindungen und erhältlich mit Hilfe des erfindungsgemäßen Verfahrens (b).

Man erhält sie beispielsweise, wenn man 5-Amino-1-aryl-pyrazole der Formel (VI),

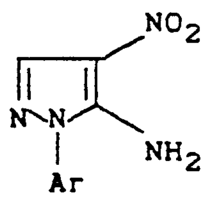

(VI)

in welcher
Ar die oben angegebene Bedeutung hat,
zunächst in einer 1. Stufe mit Acylierungsmitteln der Formel (XIV),
$R^3-\underset{\underset{O}{\|}}{C}-A^1$ (XIV)
in welcher
$R^3$ die oben angegebene Bedeutung hat und
$A^1$ für eine elektronenanziehende Abgangsgruppe wie beispielsweise Halogen oder ein Rest $R^3-\underset{\underset{O}{\|}}{C}-O$ steht
oder mit einem Isocyanat der Formel (XV),
$R^{3-1}-N=C=O$ (XV)
in welcher
$R^{3-1}$ für Alkyl oder für gegebenenfalls für substituiertes Aryl steht, vorzugsweise für Alkyl mit 1 bis 4 Kohlenstoffatomen oder für gegebenenfalls durch Fluor, Chlor, Brom, Iod, Alkyl mit 1 bis 4 Kohlenstoffatomen oder Halogenalkyl mit 1 bis 4 Kohlenstoffatomen und 1 bis 5 Halogenatomen einfach oder mehrfach, gleich oder verschieden substituiertes Phenyl steht,
gegebenenfalls in Gegenwart eines Verdünnungsmittels, wie beispielsweise Dichlormethan oder Acetonitril und gegebenenfalls in Gegenwart eines Säurebindemittels, wie beispielsweise Kaliumcarbonat oder Triethylamin in üblicher Weise bei Temperaturen zwischen -20 °C und + 120 °C acyliert und die so erhältlichen 5-Acyl-amino-pyrazole der Formel (XVI)

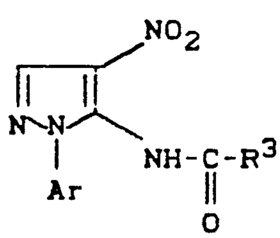

(XVI)

in welcher

$R^3$ und Ar die oben angegebene Bedeutung haben,
in einer 2. Stufe mit Alkylierungsmitteln der Formel (IV)
$R^1$-A (IV)
in welcher
$R^1$ und A die oben angegebene Bedeutung haben,
wie bei dem erfindungsgemäßen Verfahren (b) beschrieben alkyliert.

Die Acylierungsmittel der Formel (XIV) und die Alkylierungsmittel der Formel (IV) sind allgemein bekannte Verbindungen der organischen Chemie.

Die zur Durchführung des erfindungsgemäßen Verfahrens (d) als Ausgangsstoffe benötigten Pyrazolylcarbonsäure-Derivate sind durch die Formel (I e) allgemein definiert. In dieser Formel (I e) steht Ar vorzugsweise für diejenigen Reste, die bereits im Zusammenhang mit der Beschreibung der erfindungsgemäßen Stoffen der Formel (I) als bevorzugt für diesen Substituenten genannt wurden. Y steht vorzugsweise für geradkettiges oder verzweigtes Alkylen, Alkenylen oder Alkinylen mit jeweils bis zu 8 Kohlenstoffatomen, vorzugsweise für Methylen, Ethylen, Propylen oder Butylen, Propenylen oder Butenylen oder Propinylen bzw. Butinylen.

Die Pyrazolylcarbonsäure-Derivate der Formel (I e) sind erfindungsgemäße Verbindungen und erhältlich mit Hilfe des erfindungsgemäßen Verfahrens (c), insbesondere, wenn man die nach Verfahren (b) erhältlichen 5-Acylamino-4-nitro-1-aryl-pyrazole der Formel (Ic-1),

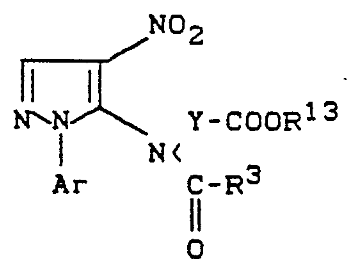

(Ic-1)

in welcher
$R^3$, Ar und Y die oben angegebene Bedeutung haben und
$R^{13}$ für Methyl oder Ethyl steht,
mit Basen wie beispielsweise wässriger Natronlauge gegebenenfalls in Gegenwart eines Verdünnungsmittels, wie beispielsweise Methanol oder Ethanol bei Temperaturen zwischen 0 °C und 120 °C gleichzeitig verseift und deacyliert.

Die zur Durchführung des erfindungsgemäßen Verfahrens (d) weiterhin als Ausgangsstoffe benötigten Alkohole, Amine oder Salze sind durch die Formel (V) allgemein definiert. In dieser Formel (V) stehen $R^4$ und Z vorzugsweise für diejenigen Reste, die bereits im Zusammenhang mit der Beschreibung der erfindungsgemäßen Stoffe der Formel (I) als bevorzugt für diese Substituenten genannt wurden.

Die Alkohole, Amine und Salze der Formel (V) sind allgemein bekannte Verbindungen der organischen Chemie.

Als Verdünnungsmittel zur Durchführung des Herstellungsverfahrens (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) kann gegebenenfalls in Gegenwart eines geeigneten Säurebindemittels durchgeführt werden. Als solche kommen alle üblichen anorganischen oder organischen Basen infragen. Hierzu gehören beispielsweise Alkalimetallhydroxide, wie Natriumhydroxid oder Kaliumhydroxid, Alkalimetallcarbonate, wie Natriumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als Reaktionspartner eingesetzten Amin der Formel (III) gleichzeitig als Säurebindemittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +200 °C, vorzugsweise bei Temperaturen zwischen 0 °C und +150 °C.

Zur Durchführung des erfindungsgemäßen Verfahrens (a) setzt man pro Mol an 5-Halogen-4-nitro-1-aryl-pyrazol der Formel (II) im allgemeinen 1,0 bis 10,0 Mol, vorzugsweise 1,0 bis 5,0 Mol an Amin der Formel (III) ein. Die Reaktionsdurchführung, Aufarbeitung und Isolierung der Reaktionsprodukte der Formel - (I) erfolgt nach allgemein üblichen Verfahren.

Als Verdünnungsmittel zur Durchführung des erfindungsgemäßen Verfahrens (b) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylen glykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (b) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natrium carbonat oder Natriumhydrogencarbonat oder auch tertäre Amine, wie beispielsweise Triethylamin, N,N-Dimethylani lin, Pyridin, 4-(N,N-Dimethylamino)pyridin, Diazabicyclooctan - (DABCO), Diazabicyclononen (DBN) oder Diazabicycloundecen (DBU).

Die Reaktionstemperaturen können bei der Durchführung des Herstellungsverfahrens (b) in einem größeren Bereich variiert werden. Im allgemeinen arbeitet man zwischen -20 °C und +150 °C, vorzugsweise zwischen 0 °C und +100 °C.

Zur Durchführung des Herstellungsverfahrens (b) setzt man pro Mol 5-Amino-4-nitro-1-aryl-pyrazol der Formel (I a) im allgemeinen 1,0 bis 20,0 Mol, vorzugsweise 1,0 bis 15,0 Mol an Alkylierungsmittel der Formel (IV) und gegebenenfalls 1,0 bis 3,0 Mol, vorzugsweise 1,0 bis 2,0 Mol an Säurebindemittel sowie 0,01 bis 1,0 Mol an Phasentransferkatalysator ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (c) kommen inerte organische oder anorganische Lösungsmittel infrage. Insbesondere seien die bei Verfahren (b) aufgezählten organischen Lösungsmittel genannt. Darüber hinaus sind Alkohole wie Methanol oder Ethanol oder deren Gemische mit Wasser besonders bevorzugt.

Das Verfahren (c) wird entweder in Gegenwart einer starken Säure wie beispielsweise Salzsäure, Trifluoressigsäure oder Bromwasserstoffsäure in Eisessig oder in Gegenwart einer Base durchgeführt. Als Basen bevorzugt sind wässrige Lösungen von Natriumhydroxid oder Kaliumhydroxid.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +150 °C, vorzugsweise bei Temperaturen zwischen 0 °C und +120 °C.

Zur Durchführung des erfindungsgemäßen Verfahrens (c) setzt man pro Mol 5-Acylamino-4-nitro-1-aryl-pyrazol der Formel (I c) im allgemeinen 1 bis 30 Mol, vorzugsweise 1 bis 15 Mol, an Säure oder Base ein. Die Reaktionsdurchführung, Aufarbeitung und Isolierung der Reaktionsprodukte der Formel (I b) erfolgt in allgemein üblicher Art und Weise.

Als Verdünnungsmittel zur Durchführung des Verfahrens (d) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder diethylether, Ketone wie Aceton oder Butanon, Nitrile, wie Acetonitril oder

Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid, Verwendet man als Reaktionspartner der Formel (V) Alkohole oder Amine in flüssiger Form, so ist es auch möglich, diese in einem entsprechenden Überschuß gleichzeitig als Verdünnungsmittel einzusetzen.

Zur Salzbildung gemäß Verfahren (d) d.h. in den Fällen, wo $R^4$ in Formel (V) für ein anorganisches oder organisches Kation steht und Z für Sauerstoff steht, kommen organische oder wässrige Lösungsmittel oder organisch-wässrige Lösungsmittelgemische in Frage. Vorzugsweise verwendet man hierbei Alkohole wie Methanol, Ethanol oder Propanol oder deren Gemische mit Wasser sowie reines Wasser als Verdünnungsmittel.

Das erfindungsgemäße Verfahren (d) wird gegebenenfalls (d.h. in den Fällen, wo eine Veresterungs- oder Amidierungsreaktion stattfindet) in Gegenwart eines geeigneten Reaktionshilfsmittels durchgeführt. Als solche kommen prinzipiell alle üblichen für Veresterungen und Amidierungen verwendbaren Reaktionshilfsmittel in Frage. Beispielhaft genannt seien Säurehalogenidbildner, wie Thionylchlorid, Phosphortrichlorid, Phosphorpentachlorid, Phosphoroxychlorid oder Aktivesterkomponenten, wie N-Hydroxy-Succinimid, Anhydridbildner wie Chlorameisensäure-4-nitrophenylester oder übliche Kondensationsmittel, wie Dicyclohexylcarbodiimid (DCC), Triphenylphosphin im Gemisch mit Tetrachlorkohlenstoff, N,N'-Carbonyl-diimidazol oder N-Ethoxycarbonyl-2-ethoxy-dihydrochinolin (EEDQ).

Das erfindungsgemäße Verfahren (d) kann gegebenenfalls in Gegenwart eines geeigneten Säurebindemittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, tertiäre Amine, wie Triethylamin, N,N-Dimethylanilin, Pyridin, N,N-Dimethylaminopyridin, Diazabicyclooctan (DABCO); Diazabicyclononen (DBN) oder Diazabicycloundecen (DBU). Auch ein entsprechender Überschuß eines gleichzeitig als Reaktionspartner der Formel - (V) verwendeten Amins oder Hydroxids kann gegebenenfalls als Säurebindemittel dienen.

Die Reaktionstemperaturen können bei der Durchführung des erfindungsgemäßen Verfahrens (d) in einem größeren Bereich variiert werden. Im allgemeinen arbeitet man bei Temperaturen zwischen -20 °C und +150 °C, vorzugsweise bei Temperaturen zwischen 0 °C und +100 °C.

Zur Durchführung des erfindungsgemäßen Verfahrens (d) setzt man pro Mol an Pyrazolylcarbonsäure-Derivat der Formel (I e) im allgemeinen 1,0 bis 20,0 Mol, vorzugsweise 1,0 bia 10,0 Mol an Alkohol, Amin oder Hydroxid der Formel (V), 1,0 bis 5,0 Mol, vorzugsweise 1,0 bis 2,0 Mol an Reaktionshilfsmittel und gegebenenfalls 1,0 bis 2,0 Mol an Säurebindemittel ein.

In den Fällen, wo eine Veresterungs-oder Amidierungsreaktion stattfindet, ist es vorteilhaft, zunächst aus den Pyrazolylcarbonsäure-Derivaten der Formel (I e) und dem Reaktionshilfsmittel nach üblichen Verfahren einen aktivierten Komplex (Säurehalogenid, Aktivester, gemischtes Säureanhydrid etc.) herzustellen, welcher gegebenenfalls isoliert werden kann und entweder in einem getrennten Reaktionsschritt oder im Eintopfverfahren mit dem Alkohol oder Amin der Formel (V) umgesetzt wird. Die Zugabe des Säurebindemittels kann dabei je nach dem verwendeten Reaktionshilfsmittel entweder in der 1. Stufe zu Bildung des aktivierten Komplexes oder in der 2. Stufe zur Umsetzung desselben nützlich sein. Die Reaktionsdurchführung, Aufarbeitung und Isolierung der Reaktionsprodukte der Formel (I d) erfolgt nach allgemein üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung mono-und dikotyler Unkräuter in monokotylen und dikotylen Kulturen wie beispielsweise Baumwolle, Gerste oder Weizen einsetzen.

In entsprechenden Aufwandmengen zeigen die erfindungsgemäßen Wirkstoffe daneben auch insektizide und fungizide Wirksamkeiten und lassen sich beispielsweise zur Bekämpfung von Blattinsekten oder von Hygiene-und Vorratsschädlingen oder zur Bekämpfung von Reiskrankheiten wie beispielsweise gegen den Erreger der Reisfleckenkrankheit (Pyricularia oryzae) einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park-und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großen Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einflüß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen-oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kabalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl) -3-methylthio-1,2,4-triazin-5(4H)-on zur Unkrautbekämpfung in Sojabohnen, in Frage.

Auch Mischungen mit N,N-Dimethyl-N'-(3-trifluormethylphenyl)-harnstoff; N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5(4H)-on; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-[4-(3,5-Dichlorpyrid-2-yloxy)-phenoxy]-propionsäure-2-benzyloxyethylester), --(trimethylsilylmethylester) oder -(2,2-diethoxyethylester); N-(1-Ethylpropyl)-3,4-dimethyl-2,6-dinitroanilin; exo-1-Methyl-4-(1-methylethyl)-2-(2-methylphenylmethoxy)-7-oxabicyclo-[2.2.1]-heptan; 2-{4-[[3-Chlor-5-(trifluormethyl)-2-pyridinyl]-oxy]-phenoxy}-propansäure bzw.-propansäureethylester; N,N-Diisopropyl-S-(2,3,3-trichlorallyl)thiolcarbamat; N-Methyl-2-(1,3-benzthiazol-2-yloxy)-acetanilid; Methyl-5-(2,4-dichlorphenoxy)-2-nitrobenzoat 3,5-Diiod-4-hydroxybenzonitril; 3-Isopropyl-2,1,3-benzothiadiazin-4-on-2,2-dioxid; 2-Chlor-N-{[[4-methoxy-6-methyl-1,3,5-triazin-2-yl)-amino]-carbonyl}-benzolsulfonamid; 4-Ethylamino-2-t-butylamino-6-methylthio-s-triazin; 2-[4-(2,4,-Dichlorphenoxy)-phenoxy-]-propionsäuremethylester oder [(4-Amino-3,5-Dichlor-6-fluor-2-pyridinyl)-oxy]-essigsäure bzw. -1-methylheptylester sind gegebenenfalls von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Anwendung als Wachstumsregulatoren können die erfindungsgemäßen Wirkstoffe in den Formulierungen ebenfalls in Mischung mit anderen bekannten Wirkstoffen vor liegen, wie Fungizide, Insektizide, Akarizide und Herbizide, sowie in Mischungen mit Düngemitteln und anderen-Wachstumsregulatoren.

Die Wirkstoffe können dabei ebenfalls als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach-dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele:

Beispiel 1:

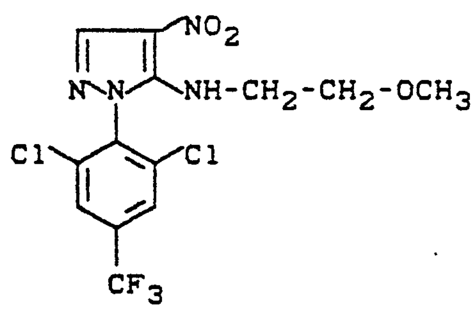

(Verfahren a)

12.0 g (0,03 Mol) 5-Brom-1-(2,6-dichlor-4-trifluormethyl phenyl)-4-nitro-pyrazol und 10 g (0,13 Mol) 2-Methoxyethylamin werden in 50 ml Dioxan 12 Stunden bei 50 °C bis 60 °C gerührt. Zur Aufarbeitung gießt man den erkalteten Reaktionsansatz in Wasser und extrahiert mehrfach mit Chloroform. Die vereinigten organischen Phasen werden mit Wasser gewaschen, über Natriumsulfat getrocknet im Vakuum eingeengt und mit Ligroin kristallisiert. Man erhält 6,6 g (55 % der Theorie) an 1-(2,6-Dichlor-4-trifluormethyl-phenyl)-5-(2-methoxyethylamino)-4-nitropyrazol vom Schmelzpunkt 85 °C -90 °C.

Herstellung der Ausgangsverbindung:

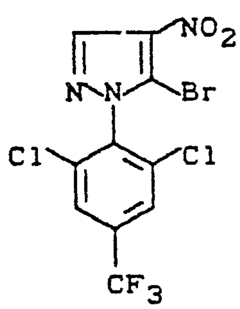

17,1 g (0,05 Mol) 5-Amino-4-nitro-1-(2,6-dichlor-4-trifluormethyl-phenyl)-pyrazol (vgl. DE-OS 3 402 308) in 75 ml Bromoform werden tropfenweise unter Rühren innerhalb von 15 Minuten mit 18 ml (0,16 Mol) t-Butylnitrit versetzt, wobei die Temperatur der Reaktionsmischung auf 50 °C ansteigt. Nach beendeter Zugabe rührt man eine weitere Stunde bei Rückflußtemperatur und engt die Mischung im Vakuum ein. Man erhält 18,0 g (89 % der Theorie) an 1-(2,6-Dichlor-4-trifluormethyl-phenyl)-4-nitro-5-brom-pyrazol vom Schmelzpunkt 96 °C.

Beispiel 2:

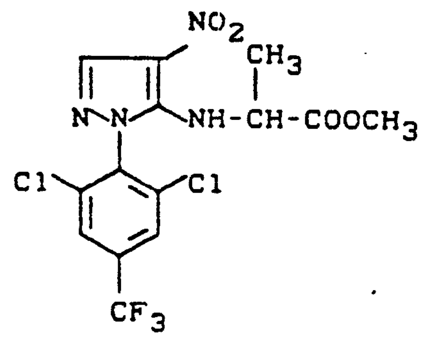

(Verfahren b)

Zu 3,4 g (0,01 Mol) 1-(2,6-Dichlor-4-trifluormethylphenyl)-4-nitro-5-amino-pyrazol gelöst in 30 ml Acetonitril gibt man 2,8 g (0,02 Mol) Kaliumcarbonat und tropft bei Raumtemperatur innerhalb von 10 Minuten 3,7 g (0,022 Mol) α-Brompropionsäuremethylester gelöst in 10 ml Acetonitril zu. Das Reaktionsgemisch wird 2 1/2 Stunden unter Rückfluß erhitzt, heiß filtriert und das Lösungsmittel sowie der überschüssige α-Brompropionester durch Einengen im Vakuum entfernt. Der Rückstand wird aus Petrolether kristallisiert. Man erhält 3,8 g (88 % der Theorie) an α-{N-[1-(2,6-Dichlor-4-trifluormethylphenyl)-4-nitro-pyrazol-5-yl]-amino}-propionsäuremethylester vom Schmelzpunkt 123 °C -130 °C.

In entsprechender Weise und gemäß den allgemeinen Angaben zur Herstellung erhält man die in der folgenden Tabelle aufgeführten 1-Aryl-4-nitro-pyrazole der Formel (I):

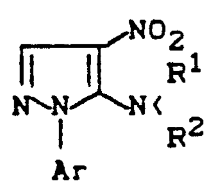

(I)

<u>Tabelle 2</u>

| Beispiel-Nr. | $R^1$ | $R^2$ | $-N\langle^{R^1}_{R_2}$ | Ar | Physikalische Eigenschaften |
|---|---|---|---|---|---|
| 3 | $-CH_2-CH_2-OCH_3$ | H | - | 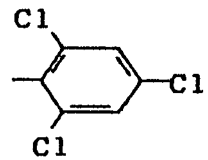 | Fp. 125° C |
| 4 | $CH_3$<br>\|<br>$-CH-COOCH_3$ | H | - | 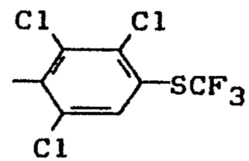 | Fp. 113° C |
| 5 | $CH_3$<br>\|<br>$-CH-COOCH_3$ | H | - | 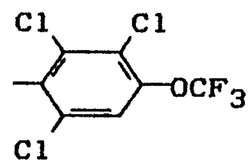 | $^1$H-NMR*):<br>δ = 1,4 (dd,3H) für<br>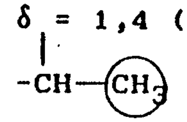<br>δ = 8,15 (s,1H) für<br>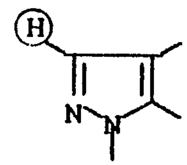 |
| 6 | $-CH_2-CH_2-OH$ | H | - | 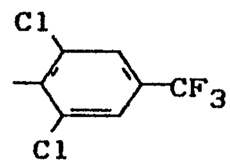 | Fp. 127-130° C |

Tabelle 2 (Fortsetzung)

| Beispiel-Nr. | $R^1$ | $R^2$ | $-N\langle^{R^1}_{R_2}$ | Ar | Physikalische Eigenschaften |
|---|---|---|---|---|---|
| 7 | $-CH_2-CH_2-N(C_2H_5)_2$ | H | - | 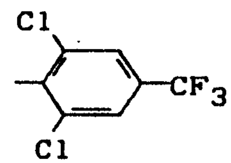 | $^1$H-NMR*) δ=1,01 (t,6H)) für 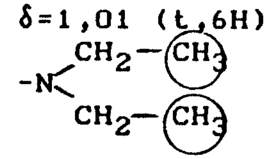 δ=8,12 (s,14) für 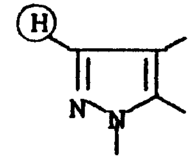 |
| 8 | $-CH_2-CH_2-CH_2-OH$ | H | - | 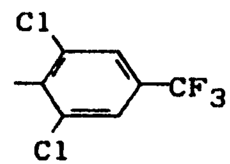 | Fp. 85-88° C |
| 9 | $-CH_2-CH_2-CH_2-OCH_3$ | H | - | 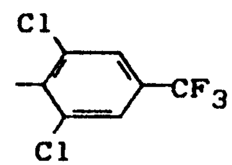 | Fp. 83° C |
| 10 | $-CH(CH_3)-COOCH_3$ | H | - | 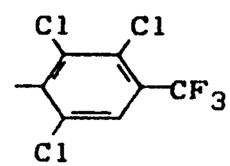 | Fp. 122-126° C |

Tabelle 2 (Fortsetzung)

| Beispiel-Nr. | $R^1$ | $R^2$ | $-N\langle^{R^1}_{R_2}$ | Ar | Physikalische Eigenschaften |
|---|---|---|---|---|---|
| 11 | - | - | 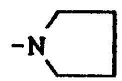 | 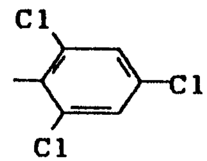 | Fp. 154° C |
| 12 | $CH_3$ | $CH_3$ | - | 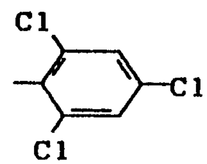 | Fp. 123° C |
| 13 | $CH_3$ | $CH_3$ | - | 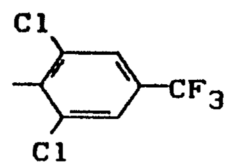 | Fp. 65-70° C |
| 14 | - | - | 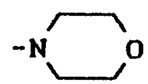 | 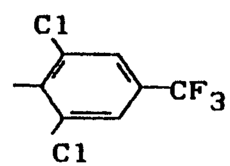 | Fp. 120-125° C |

Tabelle 2 (Fortsetzung)

| Beispiel-Nr. | $R^1$ | $R^2$ | $-N\langle{}^{R^1}_{R_2}$ | Ar | Physikalische Eigenschaften |
|---|---|---|---|---|---|
| 15 | - | - | 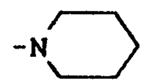 | 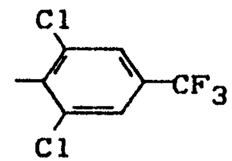 | Fp. 150-155° C |
| 16 | - | - | 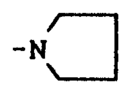 | 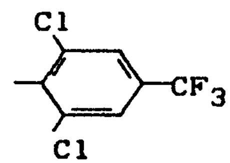 | Fp. 125-130° C |
| 17 | $-CH_2-CH=CH_2$ | H | - | 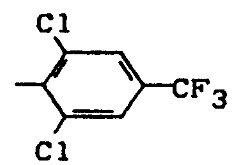 | $^1$H-NMR*) δ=3,5 (m,2H) für $-NH-CH_2-CH=CH_2$ δ=8,15 (s,1H) für 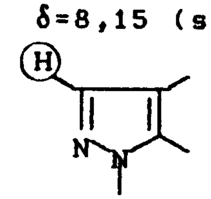 |
| 18 | $-CH_2-CH_2-CH_2-OC_2H_5$ | H | - | 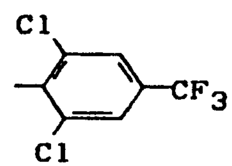 | Fp. 75-78° C |

Tabelle 2 (Fortsetzung)

| Beispiel-Nr. | $R^1$ | $R^2$ | $-N\langle{R^1 \atop R_2}$ | Ar | Physikalische Eigenschaften |
|---|---|---|---|---|---|
| 19 | $CH_3$ | $CH_3$ | - | 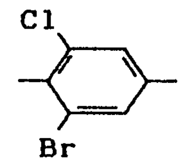 | $^1$H-NMR*)<br>$\delta$=2,8 (s,6H) für<br>$-N(CH_3)_2$<br>$\delta$=8,21 (s,1H) für<br>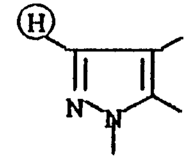 |
| 20 | $-CH_2-CH=CH_2$ | H | - | 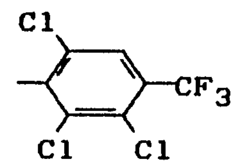 | Fp. 127-132° C |
| 21 | $CH_3$<br>\|<br>$-CH-COOC_2H_5$ | H | - | 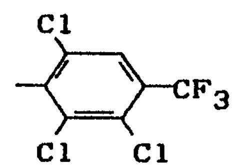 | $^1$H-NMR*)<br>$\delta$=1,4 (dd,3H) für<br>\|<br>$-CH-CH_3$<br>$\delta$=8,15 (s,1H) für<br>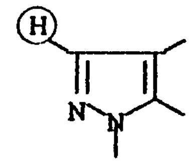 |

*) Die $^1$H-NMR-Spektren wurden in $CDCl_3$ aufgenommen mit TMS als innerem Standard. Angegeben ist die chemische Verschiebung $\delta$ in ppm.

Anwendungsbeispiele:

In den folgenden Anwendungsbeispielen wurde die nachstehend aufgeführte Verbindung als Vergleichsubstanz eingesetzt:

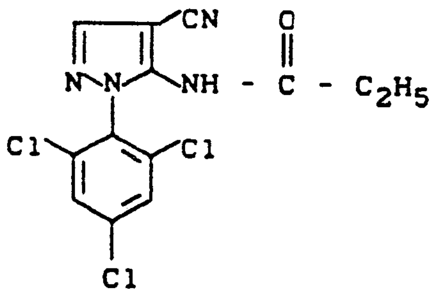

(A)

4-Cyano-5-propionamido-1-(2,4,6-trichlor-phenyl)-pyrazol (bekannt aus DE-OS 3 226 513).

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit ebenso wie in der Nutzpflanzenselektivität, insbesondere bei Weizen, Gerste und Baumwolle, gegenüber dem Stand der Technik zeigen in diesem Text z.B. die Verbindungen gemäß folgender Herstellungsbeispiele: 1 und 13.

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen boniert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der Wirksamkeit sowie in der Nutzpflanzenselektivität, insbesondere bei Weizen, gegenüber dem Stand der Technik zeigen in diesem Test z.B. die Verbindungen gemäß folgender Herstellungsbeispiele: 1, 13, 17, 20 und 21.

Beispiel C:

Entlauben und Austrocknen der Blätter bei Baumwolle

Lösungsmittel: 30 Gewichtsteile Dimethylformamid
Emulgator: 1 Gewichtsteil Poloxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach einer Woche werden Blattfall und Austrocknen der Blätter im Vergleich zur Kontrolle bonitiert. Es bedeuten:

0 kein Austrockenen der Blätter, kein Blattfall
\+ leichtes Austrocknen der Blätter, geringer Blattfall
\+ + starkes Austrocknen der Blätter, starker fall
\+ + + sehr starkes Austrocknen der Blätter, sehr starker Blattfall

Eine deutliche Überlegenheit in der Wirksamkeit gegenüber der unbehandelten Kontrolle zeigen an diesem Test beispielsweise die Verbindungen gemäß folgender Herstellungsbeispiele: 1, 6, 7 und 13

## Ansprüche

1. 1-Aryl-4-nitro-pyrazole der Formel (I)

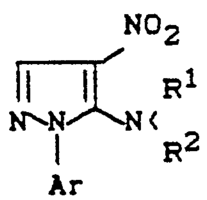

(I)

in welcher
$R^1$ für jeweils gegebenenfalls substituiertes Alkyl, Alkenyl, Alkinyl oder Cycloalkyl steht und
$R^2$ für Wasserstoff, für jeweils gegebenenfalls substituiertes Alkyl, Alkenyl, Alkinyl oder Cycloalkyl oder für einen Rest $-\underset{\underset{X}{\|}}{C}-R^3$ steht, oder
$R^1$ und $R^2$ gemeinsam mit dem Stickstoffatom, an welches sie gebunden sind, für einen gesättigten Heterocyclus stehen, der gegebenenfalls weitere Heteroatome enthalten kann und
Ar für jeweils gegebenenfalls substituiertes Phenyl oder Pyridyl steht,
wobei
X für Sauerstoff oder Schwefel steht und
$R^3$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl, für gegebenenfalls substituiertes Aryl, für gegebenenfalls substituiertes Aryloxy, für gegebenenfalls substituiertes Arylthio, oder für gegebenenfalls substituiertes Arylamino steht,
wobei jedoch
$R^2$ nur dann für Wasserstoff oder für einen Rest $-\underset{\underset{X}{\|}}{C}-R^3$ steht, wenn
$R^1$ nicht gleichzeitig für unsubstituiertes oder für durch einen Alkoximinorest substituiertes Alkyl steht.

2. 1-Aryl-4-nitro-pyrazole der Formel (I) gemäß Anspruch 1, in welcher
$R^1$ für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils infrage kommen: Halogen, Cyano, Nitro, Hydroxy, geradkettiges oder verzweigtes Alkoxy mit 1 bis 6 Kohlenstoffatomen, Amino, jeweils geradkettiges oder verzweigtes Alkylamino oder Dialkylamino mit jeweils 1 bis 6 Kohlenstoffatomen in den einzelnen Alkylteilen, Carboxy oder ein Rest $-\underset{\underset{O}{\|}}{C}-Z-R^4$;
$R^1$ weiterhin für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht,
$R^2$ für Wasserstoff, für jeweils gegebenenfalls einfach oder mehrfach, gleich oder verschieden substituiertes Alkyl, Alkenyl oder Alkinyl mit jeweils bis zu 8 Kohlenstoffatomen steht, wobei als Substituenten jeweils

infrage kommen: Halogen, Cyano, Nitro, Hydroxy, geradkettiges oder verzweigtes Alkoxy mit 1 bis 6 Kohlenstoffatomen, Amino oder jeweils geradkettiges oder verzweigtes Alkylamino oder Dialkylamino mit jeweils 1 bis 6 Kohlenstoffatomen in den einzelnen Alkylteilen; $R^2$ weiterhin für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht oder außerdem für einen Rest $-\underset{\underset{X}{\|}}{C}-R^3$ steht oder

$R^1$ und $R^2$ gemeinsam mit dem Stickstoffatom, an welches sie gebunden sind, für einen 3-bis 7-gliedrigen gesättigten Heterocyclus stehen, der bis zu 2 weitere Heteroatome, insbesondere Stickstoff, Sauerstoff und/oder Schwefel enthalten kann und

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_n-R^6$ wobei

X für Sauerstoff oder Schwefel steht,

$R^3$ für Wasserstoff, für jeweils geradkettiges oder verzweigtes Alkyl, Alkoxy, Alkoxyalkyl, Alkylthio, Alkylthioalkyl, Alkyl amino, Dialkylamino, oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen, für jeweils geradkettiges oder verzweigtes Alkenyl oder Alkinyl mit jeweils 2 bis 4 Kohlenstoffatomen, außerdem für gegebenenfalls einfach oder mehrfach, gleich oder verschieden durch Halogen, Alkyl oder Halogenalkyl mit jeweils 1 bis 4 Kohlenstoffatomen und im Fall des Halogenalkyl mit 1 bis 5 Halogenatomen substituiertes Cycloalkyl mit 3 bis 7 Kohlenstoffatomen, sowie für jeweils gegebenenfalls einfach oder mehrfach gleich oder verschieden substituiertes Phenyl, Phenoxy, Phenylthio oder Phenylamino steht, wobei als Phenylsubstituenten jeweils infrage kommen: Halogen, jeweils geradkettiges oder verzweigtes Alkyl, Alkoxy oder Halogenalkyl mit jeweils 1 bis 4 Kohlenstoffatomen und im Fall des Halogenalkyl mit 1 bis 9 gleichen oder verschiedenen Halogenatomen,

Z für Sauerstoff oder einen Rest $-\underset{|}{N}-R^5$ steht,

$R^4$ für jeweils geradkettiges oder verzweigtes Alkyl, Alkenyl oder Alkinyl mit jeweils bis zu 6 Kohlenstoffatomen steht oder für den Fall, daß Z für einen -NH-Rest oder einen Alkansulfonamido-Rest steht, auch für Wasserstoff steht oder für den Fall, daß Z für Sauerstoff steht auch für ein Äquivalent eines Alkali-, Erdalkali-, Übergangsmetall-oder eines gegebenenfalls durch Alkyl mit 1 bis 18 Kohlenstoffatomen, Benzyl oder Phenethyl substituierten Ammoniumions steht und

$R^5$ für Wasserstoff, für jeweils geradkettiges oder verzweigtes Alkyl, Alkenyl oder Alkinyl mit jeweils bis zu 6 Kohlenstoffatomen oder für jeweils geradkettiges oder verzweigtes Alkoxy oder Alkylsulfonyl mit jeweils 1 bis 6 Kohlenstoffatomen steht, oder

$R^4$ und $R^5$ gemeinsam mit dem Stickstoffatom, an welches sie gebunden sind, für einen 3-bis 7-gliedrigen gesättigten Heterocyclus stehen, der bis zu 2 weitere Heteroatome, insbesondere Stickstoff, Sauerstoff und/oder Schwefel enthalten kann,

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

n für eine Zahl 0, 1 oder 2 steht.

3. 1-Aryl-4-nitro-pyrazole der Formel (I) gemäß Anspruch 1, in welcher

$R^1$ für jeweils gegebenenfalls ein-bis fünffach, gleich oder verschieden substituiertes Methyl, Ethyl, n-oder i-Propyl, n-, i-, s-oder t Butyl, Allyl, Butenyl, Butinyl oder Propargyl steht, wobei als Substituenten jeweils infrage kommen: Fluor, Chlor, Brom, Iod, Cyano, Nitro, Hydroxy, Methoxy, Ethoxy, n-oder i-Propoxy, Amino, Methylamino, Ethylamino, Dimethylamino, Diethylamino oder Di-n-propylamino, Carboxy oder ein Rest $-\underset{\underset{O}{\|}}{C}-Z-R_4$; $R^1$ weiterhin für jeweils gegebenenfalls ein-bis fünffach, gleich oder verschieden durch Chlor, Brom, Methyl oder Ethyl substituiertes Cyclopropyl, Cyclopentyl, Cyclohexyl oder Cycloheptyl steht,

$R^2$ für Wasserstoff, für jeweils gegebenenfalls ein-bis dreifach, gleich oder verschieden substituiertes Methyl, Ethyl, n-oder i-Propyl, n-, i-, s-oder t-Butyl, Allyl, Butenyl, Propargyl oder Butinyl steht, wobei als Substituenten jeweils infrage kommen: Fluor, Chlor, Brom, Iod, Cyano, Nitro, Hydroxy, Methoxy, Ethoxy, n-oder i-Propoxy, Amino, Methylamino, Ethylamino, Dimethylamino, Diethylamino oder Di-n-propylamino; $R^2$ weiterhin für jeweils gegebenenfalls ein-bis fünffach, gleich oder verschieden durch Chlor, Brom, Methyl oder Ethyl substituiertes Cyclopropyl, Cyclopentyl, Cyclohexyl oder Cycloheptyl steht oder auch für einen Rest $-\underset{\underset{X}{\|}}{C}-R^3$ steht oder

$R^1$ und $R^2$ gemeinsam mit dem Stickstoffatom, an welches sie gebunden sind, für Pyrrolidinyl, Piperidinyl,

Perhydroazepinyl oder Morpholinyl stehen, und

Ar für gegebenenfalls ein-bis fünffach, gleich oder verschieden substituiertes Phenyl oder für gegebenenfalls ein-bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n-und i-Propyl, n-, i,-s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Di chlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_n-R^6$,

wobei

X für Sauerstoff oder Schwefel steht und

$R^3$ für Wasserstoff, Methyl, Ethyl, n-und i-Propyl, Methoy, Ethoxy, Methoxymethyl, Ethoxymethyl, Methoxyethyl, Ethoxyethyl, Methylthio, Ethylthio, Methylthiomethyl, Methylamino, Ethylamino, Dimethylamino, Allyl, Propargyl, Butenyl, Trifluormethyl, Trichlormethyl, Dichlorfluormethyl, Difluorchlormethyl, Chlormethyl, Iodmethyl, Brommethyl, Dichlormethyl, 1-Chlorethyl, 2-Chlorethyl, 2-Bromethyl, Heptafluor-n-propyl, für jeweils gegebnenfalls ein bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

Z für Sauerstoff oder einen Rest $-\underset{|}{N}-R^5$ steht,

$R^4$ für Methyl, Ethyl, n-oder i-Propyl, n-, i-, s-oder t-Butyl, Allyl oder Propargyl steht, oder für den Fall, daß Z für einen -NH-Rest oder einen Alkansulfonamido-Rest steht, auch für Wasserstoff steht oder, für den Fall, daß Z für Sauerstoff steht, auch für ein Äquivalent eines Natrium-, Kalium-, Calcium-, Eisen-, Kupfer-, Nickel-, Zinn-, Magnesium-, Zink-, Mangan-, Barium-, Cobalt-oder Ammonium-bzw. Benzylammonium-bzw. Alkylammoniumsalzes steht, wobei Alkyl für Methyl, Ethyl, n-oder i-Propyl, n-, i-, s-oder t-Butyl, n-oder i-Pentyl, n-oder i-Hexyl, n-oder i-Nonyl, n-oder i-Dodecyl steht,

$R^5$ für Wasserstoff, Methyl, Ethyl, n-oder i-Propyl, n-, i-, s-oder t-Butyl, Allyl, Propargyl, Methoxy, Ethoxy, n-oder i-Propoxy, Methylsulfonyl oder Ethylsulfonyl steht, oder

$R^4$ und $R^5$ gemeinsam mit dem Stickstoffatom, an welches sie gebunden sind, für Pyrrolidinyl, Piperidinyl, Perhydroazepinyl, Piperazinyl oder Morpholinyl stehen und $R^6$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht und

n für eine Zahl 0, 1 und 2 steht

wobei jedoch

$R^2$ nur dann für Wasserstoff oder für einen Rest $-\underset{X}{\overset{\|}{C}}-R^3$ steht, wenn $R^1$ nicht gleichzeitig für unsubstituiertes oder für durch einen Alkoximinorest substituiertes Alkyl steht.

4. 1-Aryl-4-nitro-pyrazole der Formel (I),

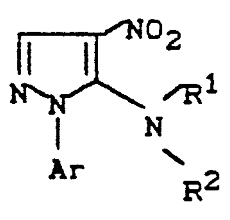

(I)

in welcher

$R^1$ für jeweils gegebenenfalls substituiertes Alkyl, Alkeny, Alkinyl oder Cycloalkyl steht und

$R^2$ für Wasserstoff, für jeweils gegebenenfalls substituiertes Alkyl, Alkenyl, Alkinyl oder Cycloalkyl oder für einen Rest- $\underset{X}{\overset{\|}{C}}-R^3$ steht, oder

$R^1$ und $R^2$ gemeinsam mit dem Stickstoffatom, an welches sie gebunden sind, für einen gesättigten Heterocyclus stehen, der gegebenenfalls weitere Heteroatome enthalten kann und

Ar für jeweils gegebenenfalls substituiertes Phenyl oder Pyridyl steht,

wobei

X für Sauerstoff oder Schwefel steht und

$R^3$ für Wasserstoff, Alkyl, Alkoxy, Alkoxyalkyl, Alkylthio, Alkylthioalkyl, Alkylamino, Dialkylamino, Halogenalkyl, Alkenyl, Alkinyl, für gegebenenfalls substituiertes Cycloalkyl, für gegebenenfalls substituiertes Aryl, für gegebenenfalls substituiertes Aryloxy, für gegebenenfalls substituiertes Arylthio, oder für gegebenenfalls substituiertes Arylamino steht,
wobei jedoch
$R^2$ nur dann für Wasserstoff oder einen Rest $-\underset{X}{\underset{\|}{C}}-R^3$ steht, wenn
$R^1$ nicht gleichzeitig für unsubstituiertes oder für durch eine Alkoximinorest substituiertes Alkyl steht,
dadurch gekennzeichnet, daß man
(a) 5-Halogen-4-nitro-1-aryl-pyrazole der Formel (II)

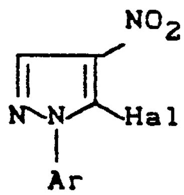

(II)

in welcher
Ar die oben angegebene Bedeutung hat und
Hal für Halogen steht,
mit Aminoverbindungen der Formel (III),

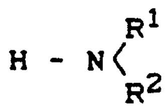

(III)

in welcher
$R^1$ und $R^2$ die oben angegebene Bedeutung haben,
gegebenenfalls in Gegenwart eines Verdünnungsmittels und gegebenenfalls in Gegenwart eines Säurebindemittels umsetzt,
oder daß man
b) 5-Amino-4-nitro-1-aryl-pyrazole der Formel (Ia),

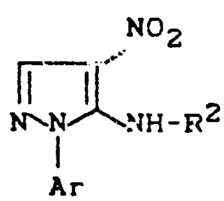

(Ia)

in welcher
$R^2$ und Ar die oben angegebene Bedeutung haben,
mit Alkylierungsmitteln der Formel (IV),
$R^1$ -A (IV)
in welcher
$R^1$ die oben angegebene Bedeutung hat und
A für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt; .
oder daß man
(c) 5-Amino-4-nitro-1-aryl-pyrazole der Formel (Ib),

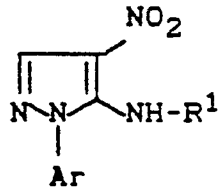

(I b)

in welcher
$R^1$ und Ar die oben angegebene Bedeutung haben,
erhält, wenn man 5-Acylamino-4-nitro-1-aryl-pyrazole der Formel (Ic),

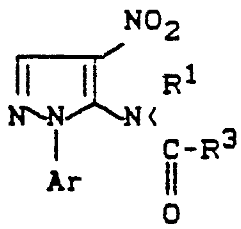

(I c)

in welcher
$R^1$, $R^3$ und Ar die oben angegebene Bedeutung haben,
mit Säuren oder Basen gegebenenfalls in Gegenwart eines Verdünnungsmittels umsetzt;
oder daß man
d) die substituierten 5-Amino-4-nitro-1-arylpyrazole der Formel (I d),

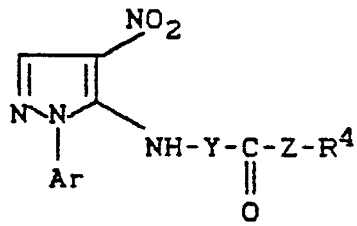

(I d)

in welcher
Ar die oben angegebene Bedeutung hat,
Y für einen zweifach verknüpften Alkylen-, Alkenylen-oder Alkinylenrest steht,
Z für Sauerstoff oder für einen Rest $-\underset{|}{N}-R^5$ steht,
$R^4$ für Alkyl, Alkenyl oder Alkinyl steht oder für den Fall, daß Z für einen -NH-Rest oder einen Alkansulfonamido-Rest steht, auch für Wasserstoff steht und für den Fall, daß Z für Sauerstoff steht auch für ein Äquivalent eines anorganischen Kations steht,
$R^5$ für Wasserstoff, Alkyl, Alkenyl, Alkinyl, Alkoxy oder für Alkylsulfonyl steht oder
$R^4$ und $R^5$ gemeinsam mit dem Stickstoffatom, an welches sie gebunden sind, für einen Heterocyclus stehen,
erhält, wenn man Pyrazolylcarbonsäure-Derivate der Formel (I e)

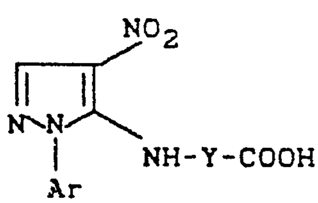

(I e)

in welcher

Ar und Y die oben angegebene Bedeutung haben,
mit Verbindungen der Formel (V)
$R^4$ - Z -H (V)
in welcher
Z und $R^4$ die oben angegebene Bedeutung haben,
gegebenenfalls in Gegenwart eines Verdünnungsmittels und gegebenenfalls in Gegenwart eines Reaktionshilfsmittels umsetzt.

5. Herbizide und pflanzenwuchsregulierende Mittel, gekennzeichnet, durch einen Gehalt an mindestens einem 1-Aryl-4-nitro-pyrazol der Formel (I) gemäß den Ansprüchen 1 bis 4.

6. Verfahren zur Bekämpfung von Unkräutern, dadurch gekennzeichnet, daß man 1-Aryl-4-nitro-pyrazole der Formel (I) gemäß den Ansprüchen 1 bis 4 auf Unkräuter und/oder ihren Lebensraum einwirken läßt.

7. Verwendung von 1-Aryl-4-nitro-pyrazolen der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man 1-Aryl-4-nitro-pyrazole der Formel (I) gemäß den Ansprüchen 1 bis 4 mit Streckmitteln und/oder oberflächenaktiven Substanzen vermischt.